# EUROPEAN PATENT SPECIFICATION

(11) **EP 0 480 293 B1**
(45) Date of publication and mention of the grant of the patent: **03.05.1995**
(21) Application number: 91116769.0
(22) Date of filing: 01.10.1991
(51) Int. Cl.: A61B 17/11, B23K 26/10

(54) **Clamp for approximating tissue sections**
Klemmvorrichtung zum Nähen von Gewebesektionen
Clamp destiné au rapprochement de sections de tissu

(30) Priority: 11.10.1990 US 595871
(43) Date of publication of application: 15.04.1992
(73) Proprietor: LaserSurge, Inc., Rochester, New York 14623 (US)
(72) Inventor: Sauer, Jude S., Rochester, NY 14620 (US)
(74) Representative: Marsh, Roy David

(56) References cited:
- EP-A- 0 330 135
- DE-B- 2 856 386
- FR-A- 2 532 220
- GB-B- 1 033 708
- US-A- 3 620 218

## Description

### BACKGROUND OF THE INVENTION

### Field of the Invention

This invention relates to tissue welding and more particularly to apparatus for holding tissue sections in apposition and compression during application of laser energy to weld the sections together. Laser welding apparatus in accordance with the pre-characterising part of claim 1 below is disclosed in EP-A-0330135.

### Description of the Related Art

US-A-4316470 discloses an approximator for anastomotic surgery which comprises two clamp holders, one of which is movable towards the other along a pair of parallel guide bars.

Conventional surgical techniques typically require the use of sutures or staples to construct surgical connections that provide functional communication between living tissue structures. These surgical connections are generally referred to as "anastomoses". Anastomoses between cylindrical, especially tubular (i.e. hollow) structures are of significant clinical importance.

The problems associated with the utilization of sutures or staples are numerous. First, it is often time consuming and technically difficult to perform anastomoses by sutures or staples. This not only results in increased costs to the patient, a factor which cannot be overlooked as the current trend of skyrocketing health care costs continues, but creates a wide discrepancy in performance between surgeons, especially between experienced and novice surgeons. Second, the use of sutures and staples also means the introduction of "foreign bodies" which can cause trauma, inflammatory and immune response, and other adverse reactions due to the actual introduction as well as the prolonged presence of these foreign materials. Inflammation can actually cause a decrease in tensile strength and bursting strength of an anastomosis. Inadequate anastomoses pose a severe health risk to the patient if the sutured tubes or organs become sufficiently weak that they separate.

In an attempt to overcome these problems associated with sutures and staples, work recently began on the use of laser energy for welding the tubular and other types of tissue. It was discovered that properly applied laser light thermally induces intrinsic tissue changes which immediately produce hermetically sealed, strong bonds between the tissue. Such laser welding may also result in increased collagen synthesis, rapid restoration of tissue function, and enhanced healing. Additionally, advantageous for younger patients, laser welding allows the growth of welded seams as body size increases.

Thus, not only does laser tissue welding avoid the adverse effects associated with introducing foreign particles, i.e. sutures and staples, into the patient's body by avoidance of needle trauma and minimization of inflammatory and immune response, but laser welding can actually optimize the strength and functional characteristics of the anastomoses.

Still further, more automated laser tissue welding advantageously does not require the skill and time-consuming labor of the surgeon which is necessary for suturing and stapling. In fact, once the equipment is properly placed by a surgeon, a nurse or technician can simply switch on the laser, which provides the light necessary to weld the tissue. This laser delivery technique requires no manual manipulation during the actual welding of the tissue and usually requires less than 10 seconds of lasing time. Thus, a mechanical means for holding tissue during laser welding also provides an increased level of consistency which simply cannot be achieved in the individualized hand-manipulated suturing and stapling techniques or in less automated laser tissue welding techniques. The automated control of the laser welding parameters, rather than the skill or experience of a particular surgeon, determines the immediate success of the welding as well as the long term holding strength of the anastomosis.

The two essential criteria for successful laser welding are: (1) control of laser energy delivery and (2) tissue apposition. Control of laser energy (precision and consistency) is important to ensure that the desired amount of incoming light energy is absorbed by the tissue. This means providing consistent laser energy density at a specific rate (i.e. fluence) over the entire anastomotic seam. Tissue apposition is critical since the ends of the hollow tubular sections to be welded must be in substantial abutment and accurate alignment to ensure that the laser energy effectively fuses the entire seam formed at the abutment. Substantial abutment also requires compression of the edges of the tubular sections. Deficient apposition can cause leakage or the formation of weak tissue bonds. Inadequate anastomses can result in separation of the tissue sections, abnormal formation of fibrous tissue (adhesions) or undesired narrowing of the passage between the tubular sections (stenosis). The importance of tissue apposition cannot be overemphasized.

To date, many failed attempts at producing an effective laser anastomosis can be attributed to inadequate tissue approximation. Such inadequate laser welds have forced surgeons to rely on the use of "stay" sutures to assist in tissue alignment and orientation during welding. Such stay sutures, usually numbering at least three for each anastomosis, are typically left at the wound site and result in all of the accompanying drawbacks and deficiencies enumerated above. Therefore, there exists a need for a way to provide precise apposition of the ends of the tubular tissue sections to be welded as well as a need for maintaining the abutting tissue ends in this position during application of laser energy to the seam.

An apparatus and method for precisely proximating the tissue to create effective laser tissue welding would have virtually limitless number of applications. For example, such apparatus could be used in reversing vasectomies (i.e. a refertilization procedure known as "vasvasostomy") by laser welding the blocked vas deferens to re-establish communication or used in fallopian tube anastomoses for reversing surgically-induced sterilization or repairing defects to help allow women to achieve desired pregnancy. This is especially significant with the current high divorce rate, and the resulting remarriages, where many men and women seek to have a second family and thus require reversal of their surgical sterilization. An apparatus and method for performing these techniques by laser welding would provide an efficient, accurate and improved way to reverse sterilization, plus an increased success rate and reduced health risks to the patient. It would also result in increased consistency and decreased surgery time. Additional uses for such laser welding with the resulting aforementioned advantages could include anastomoses for the bowel, ureters, urethra, blood vessels, biliary tissue, etc. In short, an apparatus for maintaining and securing tissue in close apposition and in correct alignment to enable accurate application of laser energy to laser weld the tissue sections would provide countless advantages over the prior surgical procedures of suturing and stapling and over the prior hand-manipulated laser tissue welding techniques.

### SUMMARY OF THE INVENTION

The distinctive features of the present invention are recited in the characterising part of claim 1.

Each clamping means preferably comprises a pair of opposed arms or jaws wherein at least one of the arms is pivotably connected to the opposing arm and is pivotable from an open position spaced from the opposing arm to a closed position overlying the opposing arm. The apparatus also preferably and advantageously has anchor means in the form of a swivable retainer mounted to one of the arms of each pair of arms for holding the opposing arms together in the closed position. The arms are preferably mounted substantially perpendicular to an axle and one of the pairs of arms slides longitudinally along said axle towards the other pair of arms to bring the tissue sections in apposition.

The laser energy transmitting means preferably comprises first and second housing sections adapted to be placed over opposing sides of the clamp wherein each of the housing sections has a plurality of light transmissive elements extending therethrough. The distal end of the elements terminates near the seam formed at the abutment of the tubular tissue sections. The proximal end of the transmissive elements is connected to an external laser source. The transmissive elements preferably extend towards the seam to simultaneously transmit laser energy radially onto substantially the entire circumferential portion of the seam.

The present specification also discloses a method for holding two sections of living tissue in close approximation for laser welding comprising clamping one section of the tissue between a first pair of jaws of a clamp, clamping another section of the tissue between a second pair of jaws of the clamp, and moving one of the pairs of jaws towards the other pair to bring the two tissue sections into abutting relationship. The step of moving one pair of jaws preferably comprises the step of sliding the pair of jaws longitudinally along an axle on which said jaws are mounted.

Sufficient laser energy is applied to the seam formed at the abutting portion of the sections to weld them together. This is achieved by placing a first housing section on one side of the clamp, placing a second housing section on the opposing side of the clamp to interfit with the first housing section, wherein each housing section contains a laser transmissive conduit. Both the housing sections and the clamp are removed after the tissue sections are laser welded.

### BRIEF DESCRIPTION OF THE DRAWINGS

The present invention will be more fully appreciated as the same becomes better understood from the following detailed description of an embodiment of the present invention when considered in connection with the accompanying drawings in which:
Figure 1 is a perspective of the clamp of the present invention showing the bottom housing section 1;
Figure 2 is a top view of the clamp of the present invention in an open position;
Figure 3 is a top view of the clamp of the present invention showing the proximal arms in the closed position;
Figure 4 is a top view of the clamp of the present invention showing both the proximal and distal arms in the closed position;
Figure 5 is a top view of the clamp of the present invention in the closed position where the distal arms have been moved longitudinally to approximate the ends of the tubular tissue sections;
Figure 6 is a top view of the clamp of the present invention in the closed position showing the bottom housing; and
Figure 7 is a top view of the clamp of Figure 6 with both the bottom and top housing sections of the laser conduit housing mounted on the clamp;
Figure 8 is a cross sectional view of the top and bottom housing sections of the laser conduit housing; and
Figure 9 is a cross sectional view taken along lines 9-9 of Figure 7.

### DETAILED DESCRIPTION OF THE PREFERRED EMBODIMENTS

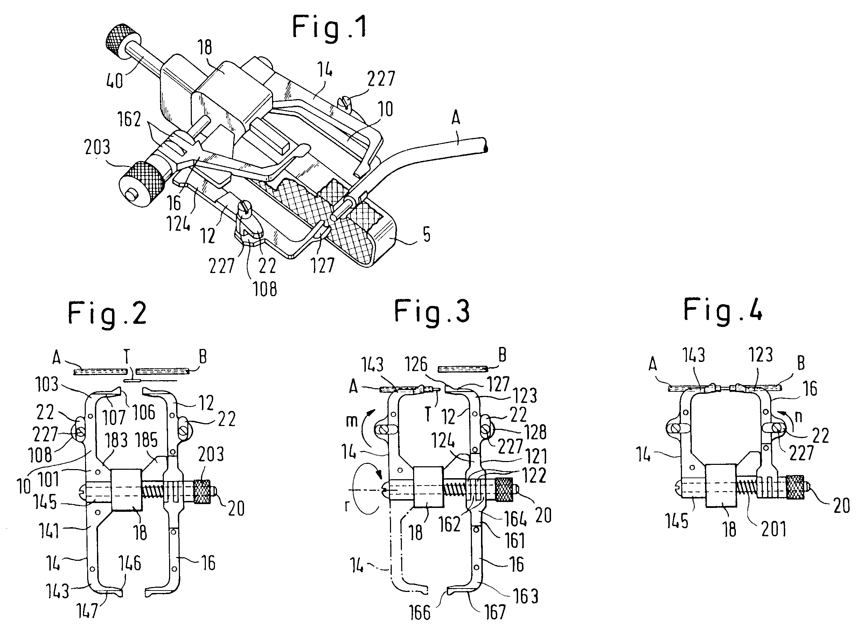

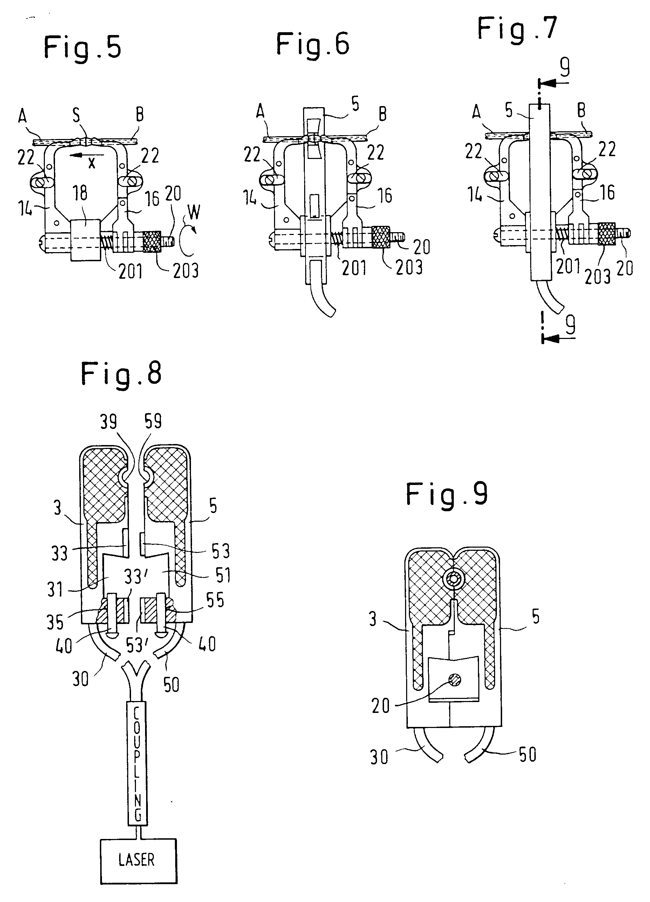

With reference now to the drawings wherein like reference numerals represent identical parts throughout the several views, and more particularly to Figure 1, reference numeral 1 represents the clamp of the present invention designed to secure two tubular tissue sections. The laser conduit housing, designated by reference numeral 2 (Figure 8), is mounted to the clamp 1 and retains the laser energy transmitting conduits which provide laser energy to weld together the tubular tissue sections grasped by the clamp 1. In the preferred embodiment, the laser housing 1 comprises a top housing section 3 and a bottom housing section 5 mounted to the upper and lower portions of the clamp 1, respectively. The housing sections 3 and 5 are together referred to as an exoscope device in U.S. Patent No. 4,892,098, the text of which is incorporated herein by reference. It should be noted that the terms "upper, lower, bottom and top" as used herein are for the readers reference since clearly, if the orientation of the clamp 1 and housing 2 changes, the designations of these terms will correspondingly change.

The clamp 1 of the present invention is designed to bring two tubular sections of tissue (e.g. a first or proximal section A and second or distal section B; see Figure 2) into abutting relationship and securely maintain these sections in this position for a sufficient amount of time for laser energy to be applied thereto to weld the two sections together. This surgical connection of such hollow tubes to establish or re-establish communication is referred to as an "anastomosis". The clamp 1 effectively provides for successful anastomosis by first firmly and independently grasping each of the tubular sections A and B and subsequently bringing these sections A, B into close apposition (abutment). The circumferential grasping of each end of the cut tissue additionally constricts the source of blood flow leading to the wounds. Laser energy can then be precisely applied around the circumference of the seam formed at the abutment to laser weld the tubular sections together. The apparatus and method for achieving this sutureless and staple-less anastomosis will now be described in detail.

Turning now to Figures 2 and 3, clamp 1 has a pair of proximal arms (jaws) comprising a lower proximal arm 10 and an upper proximal arm 14 and a pair of distal arms (jaws) comprising a lower distal arm 12 and an upper distal arm 16. (The terms "proximal", "distal", "lower" and "upper" are used for the readers reference to differentiate between the arms. Clearly, if the orientation of the clamp changes, these designations will also change.) Each pair of arms is mounted on an axle 20, preferably threaded, for pivotal movement. Axle 20 extends through an aperture in a center post 18 of the base of the clamp. An anchor or retainer 22 is mounted on the lower proximal arm 14 and on the lower distal arm 16 to secure the upper arms 14 and 16 to their respective lower arms 10, 12 in a manner described below.

The lower proximal arm 10 as shown is L-shaped with a rear end 101 connected to center post 18 and an inwardly extending free front end 103. Front end 103 has a longitudinal slot 107 formed in its top surface which preferably decreases in width at a tip portion 106. Ear 108 extends outwardly from a central portion of lower proximal arm 14 and has an aperture formed therethrough to receive a fastener to mount anchor 22. Lower proximal arm 10 is preferably rigidly mounted to axle 20 and to center post 18 so pivotal movement is prevented. However, alternately, lower proximal arm 10 could be pivotally mounted to the axle 20 to allow free rotation.

With continued reference to Figures 2 and 3, upper proximal arm 14 is pivotally mounted to axle 20 for pivotal movement with respect to lower proximal arm 10. The upper proximal arm 14 pivots between an open position spaced apart from lower proximal arm 10 (Figure 2) to a closed position overlying lower proximal arm 10 (Figure 3). Figure 3 shows upper proximal arm 14 in dotted line in the open position. In the closed position, portions of the lower surface of upper proximal arm 14 may abut portions of the upper surface of lower proximal arm 10, and preferably, at least the central portions of arms 10 and 14 abut one another in the closed position. A rear end 141 of upper proximal arm 14 includes a tubular bracket 145 which encircles axle 20 for mounting thereon. In one embodiment, upper proximal arm 14 can pivot to an open position up to an arc of approximately 270°; however, a smaller or full 360° pivot is also within the scope of the present invention. Upper proximal arm 14 has an inwardly extending free front end 143 with a longitudinal recess 147 formed in its bottom surface which preferably has a decreased width at tip portion 146, thereby cooperating with free front end 103 of lower proximal arm 10 when in the closed position. That is, recess 147 of upper proximal arm 14 cooperates with longitudinal slot 107 of the top surface of lower proximal arm 10 to form a channel therebetween to receive tubular section A when the proximal arms 14, 10 are in their closed position. Tip portions 146 and 106 of upper and lower proximal arms 14, 10, respectively, have rounded ends which form a narrow circular opening through which tubular section A extends for reasons which will become apparent from the discussion below.

Referring now to Figure 3, (for clarity, the distal arms 12 and 16 are not labelled in detail in Figure 2) lower distal arm 12 is pivotally mounted on axle 20 and is illustratively rotatable around an arc of 360°, although other arms are also contemplated. Rear end 121 of lower distal arm 12 has a pair of spaced apart clasps 122 having annular holes to receive axle 20 for mounting thereon. Rear end 121 further includes a recessed surface 124 (on the upper surface of lower distal arm 12) for reasons discussed below. A free front end 123 is substantially identical in configuration to the front end 103 of the lower proximal arm 10 as it has a longitudinal slot 127 in its upper surface preferably terminating in a decreased width portion at tip section 126. Lower distal arm 12 also includes, similar to lower proximal arm 10, an outwardly extending ear 128 having an aperture to receive a fastener for mounting anchor 22.

Upper distal arm 16 is pivotally mounted to lower distal arm 12 for movement between an open position spaced from lower distal arm 12 to a closed position overlying lower distal arm 12. Preferably upper distal arm 16 is pivotable to an arc of approximately 270°; however, clearly upper distal arm 12 can be pivotable along a larger or smaller arc. Upper distal arm 16 includes a rear end 161 having a pair of spaced apart clasps 162 with annular holes through which axle 20 extends for mounting thereon. These clasps 162 are disposed between clasps 122 of lower distal arm 12 as shown in Figure 3. Front end 163 of upper distal arm 16 is substantially identical to the front end 143 of upper proximal arm 14 in that it includes an inwardly extending portion having a longitudinal recess 167 formed in its bottom surface terminating at tip portion 166 where it preferably has an decreased width. When the arms 16, 12 are closed, longitudinal recess 167 of upper distal arm 16 cooperates with longtiudinal recess 127 of lower distal arm 12 to form a channel for receiving tubular tissue section B. Tissue section B protrudes beyond the annular opening formed at the end of cooperating tip portions 126, 166. Upper distal arm 16 also has a recessed portion 164 in its bottom surface in rear end 161.

The base of the clamp includes a central post 18, shown in the drawings shaped as a block, which has a central aperture extending therethrough to receive axle 20. As shown in Figure 2, the base further includes a distal support 185 and a proximal ear 183 preferably integral with the post 18. Distal support 185 fits within recesses 124 and 164 of lower distal arm 12 and upper distal arm 16, respectively, when the closed distal arms 12, 16 are moved inwardly towards the proximal arms 10, 14 in a manner described below. The top portion 180 (Figure 5) of the post 18 is adapted to receive top housing section 5 and the bottom portion of post 18 (not shown in the drawings) is adapted to receive the bottom housing section 3.

Axle 20, which is illustratively formed as a threaded screw, extends through the aperture in post 18 and has a spring 201 mounted thereon which is disposed intermediate clasp 122 and post 18. A longitudinal adjustment nut or cap 203 fits over the end of the axis screw 20 and is adapted to be brought into contact with clasp 162 (or alternately clasp 122) of upper distal arm 16, thereby functioning to move closed distal arms 12, 16 towards proximal arms 10, 14 in a manner described in detail below.

An anchor 22 is swivably mounted to both ear 108 of lower proximal arm 10 and ear 128 of lower distal arm 12 by a screw 227 (See Figures 2-4). Screw 227 extends through an opening formed in the anchor 22 and through the aligned aperture of the respective ear 108, 128. The anchor 22 is illustratively L-shaped in configuration and is pivotable in the direction of arrows m and n (Figures 3 and 4) from a resting position substantially parallel to the longitudinal axis of its respective arm 10, 12 to a locking position substantially perpendicular to the longitudinal axis. The anchor 22 has an undercut portion which provides a sufficient gap to receive the respective upper arm 14 and 16 as described below. A conventional nut (not shown) can optionally be mounted to the bottom end of screw 227. The anchor 22 in its locking position compresses the bottom surface of the undercut against the upper surface of the upper arms 14, 16 in the closed position.) Clearly, other means for anchoring the pair of proximal arms and pair of distal arms (e.g. snaps or screws) can be utilized as long as it provides firm securement of the arms.

Turning now to the top housing section 3 and bottom housing section 5 of laser housing 2, these housing sections are mounted to opposing sides of clamp 1 and provide the means for applying laser energy to the seam formed between abutting tubular sections A and B. As noted above, the terms "top" and "bottom" are used for convenience to denote placement on opposing sides of the clamp 1 since the clamp can be re-oriented. As shown in Figure 8, top housing section 3 includes a central recess 31 to receive the top portion of center post 18, and front and rear longitudinal slots 33', 33' formed on its inner surface. An upper channel 35 extending from the rear of housing section 3 to recess 31 provides for passage of a tightening screw 40. The tightening screw 40 is adapted to be rotated inwardly to press against central post 18 when housing section 3 is mounted thereon to effectively secure the top housing section 3 to clamp 1. A separate channel is spaced apart from upper channel 35 to allow passage of the laser transmitting conduits in channel 30.

Bottom housing section 5 is substantially identical in configuration to top housing section 3 except it is provided with projections 53, 53' on its inner surface instead of longitudinal slots 33, 33'. The projections 53, 53' are adapted to engage slots 33, 33', respectively, of top housing section 3 when bottom housing section 5 is fitted over top portion 180 of post 18 to secure the two housing sections together. Of course, alternate ways to connect the two housing halves can be utilized. Similar to top housing section 3, bottom housing section 5 includes a channel 55 to receive a screw 40 for tightening the housing section 5 against central post 18. A separate channel spaced from channel 55 receives a conduit 50 for transmitting laser light.

Both housing sections 3, 5 include a semicircular recessed portion 39, 59, respectively, through which the laser light is transmitted. The recesses 39 and 59 cooperate to form a circular enclosure for the annular seam S of the abutting tubular sections A and B when the housing sections 3, 5 are mounted to opposing sides of clamp 1 and projections 53, 53' are interfitted within longitudinal slots 33, 33'. Although the housing 2 is illustrated as comprising two discrete sections 3 and 5, clearly a single housing with hinged sections could alternatively be provided.

The channels 30, 50 for the laser conduits extends through both bottom and top housing sections 3, 5, and in a preferred embodiment comprise a series of multiple fibers radially directing light with respect to semicircular recessed portions 39 and 59 in order to transmit laser light simultaneously along the entire circumference of the seam S formed at the abutment of tubular sections A and B. The fibers preferably terminate before recesses 39, 59 so they are adjacent, but not contiguous, to the seam S. Light can travel radially to directly lase the abutted tissue or alternately a lens system such as a prism or mirror could be positioned at an angle to the seam so that incoming laser light will be directed by the lens system onto the seam. With continued reference to Figure 8, the conduits 30, 50, each containing multiple optical fibers, extend rearwardly from the housing sections 3, 5, to a coupler where the proximal ends of the multiple fibers interface with light emitted from a single standard optical fiber from a laser or are coupled directly to the laser itself. Block diagrams here illustrate coupling to a single optical fiber from an external source of laser energy. The aspects of generating the laser energy from the external source are known in the art and beyond the scope of the present invention as is the coupling of a single fiber to a laser.

With reference to Figures 2-7, the operation of the clamp 1 of the present invention will now be described. Figure 2 shows clamp 1 with both upper distal arm 14 and upper proximal arm 16 in the open position. Note that anchor 22 is disposed along ear 108 in a position substantially parallel to the longitudinal axis of lower proximal arm 10 to avoid interference with movement of upper proximal arm 14 to the closed position. As shown in Figure 3, tubular tissue section (proximal section) A is placed longitudinally in the slot 107 of lower proximal arm 10 and the upper proximal arm 14 is then pivoted in the direction of arrow r to its closed position to overlie lower proximal arm 10. Tubular section A is thus firmly fitted within the channel formed between cooperating slot 107 of lower proximal arm 10 and recess 147 of upper proximal arm 14. Anchor 22 is then rotated in the direction of arrow m to its locking position, substantially perpendicular to the longitudinal axis of the lower proximal arm 10, so that it holds the proximal arms 10, 14 together in their closed position. Proximal arms 10 and 12 provide sufficient pressure to effectively function to reduce or even stop the bleeding.

Once tubular tissue section A is securely clamped by proximal arms 10, 14, the other tubular section (distal section) B is placed within slot 127 of lower distal arm 12 and upper distal arm 16 is pivoted to its closed position to overlie lower distal arm 12 (Figure 4). Anchor 22, attached to ear 128 of lower distal arm 12, is subsequently rotated (see arrow n of Figure 4) to its perpendicular locking position to secure the distal arms 10, 16 together to thereby firmly grasp tubular section B within the channel formed between the cooperating slot 127 of lower distal arm 12 and recess 167 of upper distal arm 16. Distal arms 12 and 16 also function to reduce or stop the bleeding.

After securement of both the proximal and distal tubular sections A, B, in their respective arms, adjustment nut 203 on threaded axle 20 is rotated clockwise (in the direction of arrow w of Figure 5). This rotation moves nut 203 in the direction of arrow x to abut clasp 162. Continued rotation of abutting nut 203 thus moves distal arms 12, 16 longitudinally along axle 20 toward the proximal arms 10, 14 in the direction of arrow x, thereby compressing spring 201. Longitudinal movement of distal arms 12, 16 carries the grasped tubular section B in the same direction to bring it into apposition (abutment) with tubular section A to form seam S. Apposition preferably requires that the edges of sections A and B are compressed. Achievement of this close apposition and the ability of the clamp 1 of the present invention to hold these tubular sections in abutment enables effective anastomosis of these tubular sections when laser energy is applied to the seam.

After approximation of the tubular sections A, B by the arms of clamp 1, the bottom housing section 5 is positioned so that the seam S lies in correct alignment for tissue welding. Recess 31 of lower housing section 3 is fitted over a bottom portion of center post 18 of clamp 1 (Figure 6) and secured in position by turning the screw 40 at the back of the housing section 3 to tighten it against post 18. Recess 39 thus encloses half of the circular seam S. The upper housing section 3 is then placed over clamp 1 so that recess 51 is fitted over the top portion 180 of post 18 and projections 53, 53' engage longitudinal slots 33, 33' respectively, of lower housing portion 3. Recess 59 thereby encloses the other half of circular seam S. Similar to bottom housing section 5, top housing section 3 is locked into position by tightening screw 40 against post 18. Positioning of the housing portions 3 and 5 as thus described ensure that the seam S is fully encircled to provide precise delivery of laser light.

After interfitting the housing sections 3, 5, the external laser energy source is operated to transmit a selected dosage of laser light for a predetermined time period. The laser light travels in multiple fibers through conduits 30 and 50 and light is delivered radially to thereby apply laser energy directly and simultaneously around substantially the entire circumferential seam S formed at the abutment of tubular sections A and B. The laser energy thereby functions to weld tubular sections A and B together along seam S to provide an effective and improved anastomosis.

While the exact physiologic mechanism of the laser weld is not fully understood, tissue welding is achieved through the controlled application of light energy to the anastomastic site to produce a uniform thermal effect which causes the two sides to bind together. That is, the laser energy thermally induces intrinsic tissue changes (e.g. alterations in tissue collagen and other acellular proteins) as the electromagnetic energy from the laser is converted into thermal energy which lead to strong bonds between tissue.

Although any laser light transmissible through the conduits could be used, in a preferred embodiment where the vas deferens are to be welded, the Neodymium Yttrium Aluminum Garnet (Nd:YAG) laser such as Sharplen 2100 Nd:Yag laser or a Cooper Model 8000 Nd:Yag laser is utilized with readily available quartz optical fibers. In this embodiment, the line width of the circumferential welding light can be as narrow as 75 microns. For example, in recent experimental trials of this equipment for welding rabbit vas deferens anastomoses, five watts of power from a Nd:Yag laser operating at a continuous mode was applied for 1.5 seconds to successfully provide a uniform circumferential weld at the seam. However, it is clearly within the scope of the present invention to utilize laser energy of different densities and for different time periods to achieve the laser welding of the tubular sections. Such variations may depend on for example the type of tissue being welded, the physiologic condition of that tissue, and/or wave length of light used.

After application of sufficient laser energy to weld the tissue sections A, B, the screw 40 is loosened and the top housing section 5 is removed from clamp 1, followed by loosening of the screw 40 to remove the bottom housing section 3. The tubular section B is then removed from distal arms 12, 16 of clamp 1 pivoting anchor 22 to its longitudinal resting position, to free upper distal arm 16. Distal arm 16 is then pivoted away from tubular section B and lower distal arm 12, followed by removal of lower distal arm 12 from tubular Section B. The upper proximal arm 14 is likewise disengaged from the lower proximal arm 10 by rotating anchor 22 to its longitudinal resting position. Upper proximal arm 14 is then pivoted to its open position. Tubular section A is then removed from lower proximal arm 10.

To facilitate securement and approximation of tubular tissue sections, an absorbable stent such as that illustrated in Figure 1 and designated by reference letter T can optionally be utilized. The stent can be composed of materials which are water soluble and biocompatible such as polyvinyl alcohol (PVA) or polyvinyl pyrrolidine (PVA(PVP)). The proximal end of the stent T is inserted into the proximal tubular section A prior to clamping of the proximal arms 10, 14 around tubular section A. After closing and securement of proximal arm 14 by anchor 22, the distal tubular section B is then pulled over the distal end of the stent T. Subsequently, upper distal arm 16 is pivoted to its closed position. The stent T is preferably water soluble so it can advantageously be left in the body after removal of the clamp 1 and will be fully excreted by the body. Likewise, non-soluble stents (e.g. stainless steel wire, teflon, etc.) can be used to assist in apposition and can be removed after welding through natural occuring openings like the fimbriated end of a fallopian tube or the anus or through a small incision in other structures like the vas deferens. Other means can be provided to facilitate or enhance securement and approximation of the tubular tissue sections such as flat surfaces for compressing tissue, teeth or barbs or vacuum parts along the jaws where the tissue is held.

Optionally, a dye substance such as India Ink can be applied to the proximal and distal tubular sections A, B adjacent the seam to act as an exogenous chromophore to increase laser energy absorption into the anastomotic edges, thereby enhancing tissue welds and minimizing collateral thermal damage. This advantageously allows use of reduced power settings. Other exogenous chromophores such as Indocyanine Green dye, flourescein or endogenous chromophores such as blood or can alternately be utilized to facilitate absorption of laser energy by localizing the laser energy on the welding site.

The clamp 1 of the present invention can be used in a variety of applications for laser welding of tubular sections of the body. For example, the clamp can be used for anastomosis of the vas deferens in performing a vasovasostomy (a reversal of a vasectomy). In this instance, the proximal and vas deferens are secured within the jaws (arms) of clamp 1, brought into apposition and laser welded to re-establish communication. The clamp 1 can also be used for laser welding the fallopian tubes to reverse surgically induced sterility or repair defects. Other uses include the clamping and subsequent laser welding of the ureter, urethra, blood vessels, biliary tissue and other tubular tissue structures. Additionally, the clamp 1 can clearly be used to grasp and bring into opposition other tissue sections that are not hollow. An additional advantage of this apparatus and method for mechanically holding tissue during laser welding is that it can be used at distant less accessible sites within body cavities as is necessary in minimally invasive endoscopic or laproscopic surgical procedures.

The apparatus of the present invention can also be used to clamp metal or plastic tubes or prosthetic materials such as Goretex™ or Dacron™ for surgical uses.

In brief, therefore, by means of apparatus according to the invention, the following steps are carried out: clamping a first tissue section in a clamp; clamping a second tissue section in the clamp; moving one of the clamped tissue sections into abutting relationship with the other tissue section; applying laser energy to the seam formed at the abutting portion of the tissue sections to weld them together; and removing the clamp after the tissue sections are welded together.

## Claims

1. Surgical apparatus for laser welding the abutting ends of two hollow tubular tissue body sections (A, B), the apparatus comprising means (T) to approximate the first and second tissue section ends and means (30, 50) for transmitting laser energy to the seam to weld together the first and second tissue section ends, the apparatus being characterised in that the approximating means comprises:
first clamping means (12, 16) defining a first channel (107, 147) for accommodating and circumferentially grasping the first tissue section; and
second clamping means (10, 14) defining a second channel (127, 167) for accommodating and circumferentially grasping the second tissue section; and
means (203) carrying both the first clamping means and the second clamping means, for moving one of said clamping means toward the other clamping means to bring the first and second tissue section ends into abutting compressive relationship to form an annular seam.

2. Surgical apparatus as claimed in claim 1, wherein said means for clamping said first tissue section comprises a first pair of opposed arms (12, 16) and said means for clamping said second tissue section comprises a second pair of opposed arms (10, 14), at least one of said arms of each said pair being pivotably connected to said opposing arm and movable from an open position spaced from said opposing arm to a closed position overlying said opposing arm.

3. Surgical apparatus as claimed in claim 2, further comprising an axle (20) and a cap (203) mounted to an end of said axle, said arms being mounted substantially perpendicular to said axle, wherein rotation of said cap slides one of said pairs of arms longitudinally along said axle towards said other pair of arms.

4. Surgical apparatus as claimed in claim 3, wherein one arm of said first pair of arms if fixedly secured to said axle and both arms of said second pair of arms are pivotably mounted to said axle, said axle being aligned substantially parallel to a longitudinal axis of said clamped sections to be welded.

5. Surgical apparatus as claimed in any one of the preceding claims, further comprising anchor means (22) for holding said opposing arms of each said pair of arms in said closed position.

6. Surgical apparatus as claimed in claim 5, wherein said anchor means comprises a retainer swivably mounted to one of said arms of each said pair of arms, said retainer having an undercut portion adapted to overlie said opposing arm in said closed position, and further comprising a fastener for tightening said retainer against said arms to secure said opposing arms.

7. Surgical apparatus as claimed in any one of the preceding claims, wherein each of said opposing arms has an inwardly directed portion (107), each said portion of each said arm having a recess (147) formed therein to cooperate with said inwardly directed portion of said opposing arm when said arms are in the closed position to thereby form a channel to receive the respective tissue section.

8. Surgical apparatus as claimed in any one of the preceding claims, wherein said laser energy transmitting means comprises a housing (1) adapted to be placed over said clamping means, said housing having a plurality of light transmissive elements (30, 50) disposed therein, said distal end of said light transmissive elements terminating near said seam and said proximal end of said light transmissive elements being adapted for connection to an external laser source.

9. Surgical apparatus as claimed in claim 8, wherein said housing comprises a first housing section (3) adapted to be placed over one surface of said clamping means and a second housing section (5) adapted to be placed over an opposing surface of said clamping means.

10. Surgical apparatus as claimed in claim 8 or 9, wherein said light transmissive elements extend towards said seam in an arrangement which is adapted simultaneously to transmit laser energy to substantially the entire circumference of the seam.

11. Surgical apparatus as claimed in any one of the preceding claims, wherein said arms are mounted on an axle (20), and further comprising a center post (18) disposed on said axle intermediate said pairs of arms, and means for tightening said first and second housing sections against said center post, said tightening means comprises a screw extending through an opening in each said housing section wherein rotation of said screw moves said screw inwardly towards said center post to press against said post.

12. Surgical apparatus is claimed in any one of the preceding claims, including a stent for placement inside the first tissue section before clamping the first section, and inside the second tissue section before clamping the second section.

13. Surgical apparatus as claimed in claim 12, wherein the stent is comprised of a bioabsorbable material so that it may remain in the welded tissue sections after removal of the clamp.

14. Surgical apparatus as claimed in any one of the preceding claims, wherein each of said arms is mounted substantially perpendicular to an axle, and one of said pair of arms is slidable towards the other pair of arms longitudinally along the axle to move one of the clamped tissue sections relative to the other.

## Patentansprüche

1. Chirurgisches Instrument zum Laserschweißen der aneinander anliegenden Enden von zwei hohlen röhrenförmigen Körpergewebeabschnitten (A, B), wobei das Instrument eine Einrichtung (1) umfaßt, um die Enden des ersten und zweiten Gewebeabschnitts einander anzunähern, sowie eine Einrichtung (30, 50) zur Übertragung von Laserenergie zur Naht, um die Enden des ersten und zweiten Gewebeabschnitts zusammenzuschweißen, wobei das Instrument dadurch gekennzeichnet ist, daß die Annäherungseinrichtung umfaßt:
eine erste Klemmeinrichtung (12, 16), die einen ersten Kanal (107, 147) begrenzt, um den ersten Gewebeabschnitt aufzunehmen und in Umfangsrichtung zu fassen; und
eine zweite Klemmeinrichtung (10, 14), die einen zweiten Kanal (127, 167) begrenzt, um den zweiten Gewebeabschnitt aufzunehmen und in Umfangsrichtung zu fassen; und
eine sowohl die erste Klemmeinrichtung und die zweite Klemmeinrichtung tragende Einrichtung (203) zum Bewegen von einer der besagten Klemmeinrichtungen in Richtung der anderen Klemmeinrichtung, um zur Bildung einer ringförmigen Naht die Enden des ersten und zweiten Gewebeabschnitts in eine Druck-Anlagebeziehung zu bringen.

2. Chirurgisches Instrument nach Anspruch 1, dadurch gekennzeichnet, daß die besagte Einrichtung zum Festklemmen des besagten ersten Gewebeabschnitts ein erstes Paar von entgegengesetzten Armen (12, 16) umfaßt, und die besagte Einrichtung zum Festklemmen des besagten zweiten Gewebeabschnitts ein zweites Paar von entgegengesetzten Armen (10, 14) umfaßt, wobei mindestens einer der besagten Arme jedes besagten Paars schwenkbar mit dem besagten entgegengesetzten Arm verbunden und sich aus einer im Abstand von dem besagten entgegengesetzten Arm angeordneten offenen Stellung in eine über dem besagten entgegengesetzten Arm liegende geschlossene Stellung bewegen läßt.

3. Chirurgisches Instrument nach Anspruch 2, dadurch gekennzeichnet, daß es weiter einen Achsbolzen (20) und eine an einem Ende des besagten Achsbolzens angebrachte Kappe (203) umfaßt, wobei die besagten Arme im wesentlichen senkrecht an dem besagten Achsbolzen angebracht sind, wobei eine Drehung der besagten Kappe eines der besagten Armpaare in Längsrichtung an dem besagten Achsbolzen entlang auf das besagte andere Armpaar zu verschiebt.

4. Chirurgisches Instrument nach Anspruch 3, dadurch gekennzeichnet, daß ein Arm des besagten ersten Armpaars starr an dem besagten Achsbolzen befestigt ist, und beide Arme des besagten zweiten Armpaars schwenkbar an dem besagten Achsbolzen angebracht sind, wobei der besagte Achsbolzen im wesentlichen parallel zu einer Längsachse der besagten festgeklemmten Abschnitte ausgerichtet ist, die verschweißt werden sollen.

5. Chirurgisches Instrument nach einem beliebigen der vorangehenden Ansprüche, dadurch gekennzeichnet, daß es weiter eine Verankerungseinrichtung (22) umfaßt, um die besagten entgegengesetzten Arme jedes besagten Armpaars in der besagten geschlossenen Stellung zu halten.

6. Chirurgisches Instrument nach Anspruch 5, dadurch gekennzeichnet, daß die besagte Verankerungseinrichtung einen Halter umfaßt, der drehbar an einem der besagten Arme jedes besagten Armpaars angebracht ist, wobei der besagte Halter ein hinterschnittenes Teilstück aufweist, das so angepaßt ist, daß es in der besagten geschlossenen Stellung über dem besagten entgegengesetzten Arm liegt, und weiter eine Befestigungsvorrichtung zum Festziehen des besagten Halters gegen die besagte Arme umfaßt, um die besagten entgegengesetzten Arme festzuhalten.

7. Chirurgisches Instrument nach einem beliebigen der vorangehenden Ansprüche, dadurch gekennzeichnet, daß jeder der besagten entgegengesetzten Arme einen nach innen gerichteten Teil (103) aufweist, wobei jeder besagte Teil jedes besagten Arms eine darin ausgebildete Ausnehmung (147) aufweist, um mit dem besagten nach innen gerichteten Teil des besagten entgegengesetzten Arms zusammenzuwirken, wenn die besagten Arme in der geschlossenen Stellung sind, um dadurch einen Kanal zur Aufnahme des jeweiligen Gewebeabschnitts zu bilden.

8. Chirurgisches Instrument nach einem beliebigen der vorangehenden Ansprüche, dadurch gekennzeichnet, daß die besagte Laserenergie-Übertragungseinrichtung ein Gehäuse (1) umfaßt, das zum Plazieren über der besagten Klemmeinrichtung angepaßt ist, wobei das besagte Gehäuse eine Mehrzahl von darin angeordneten lichtdurchlässigen Elementen (30, 50) aufweist, wobei das besagte distale Ende der besagten lichtdurchlässigen Elemente in der Nähe der besagten Naht endet, und das besagte proximale Ende der besagten lichtdurchlässigen Elemente zum Verbinden mit einer äußeren Laserquelle angepaßt ist.

9. Chirurgisches Instrument nach Anspruch 8, dadurch gekennzeichnet, daß das besagte Gehäuse einen ersten Gehäuseabschnitt (3) umfaßt, der zum Plazieren über einer Oberfläche der besagten Klemmeinrichtung angepaßt ist, und einen zweiten Gehäuseabschnitt (5), der zum Plazieren über einer entgegengesetzten Oberfläche der besagten Klemmeinrichtung angepaßt ist.

10. Chirurgisches Instrument nach Anspruch 8 oder 9, dadurch gekennzeichnet, daß die besagten lichtdurchlässigen Elemente in einer Anordnung auf die besagte Naht zu verlaufen, welche für ein gleichzeitiges Übertragen von Laserenergie auf im wesentlichen den gesamten Umfang der Naht angepaßt ist.

11. Chirurgisches Instrument nach einem beliebigen der vorangehenden Ansprüche, dadurch gekennzeichnet, daß die besagten Arme auf einem Achsbolzen (20) angebracht sind, und weiter umfassend eine zwischen den besagten Armpaaren auf dem besagten Achsbolzen angeordneten Mittenstütze (18) und eine Einrichtung zum Verspannen des besagten ersten und zweiten Gehäuseabschnitts gegen die besagte Mittenstütze, wobei die besagte Spanneinrichtung eine Schraube umfaßt, die sich jeweils durch eine Öffnung in dem besagten Gehäuseabschnitt erstreckt, wobei eine Drehung der besagten Schraube die besagte Schraube nach innen in Richtung der besagten Mittenstütze bewegt, so daß sie gegen die besagte Stütze drückt.

12. Chirurgisches Instrument nach einem beliebigen der vorangehenden Ansprüche, dadurch gekennzeichnet, daß es einen Stent einschließt, für eine Plazierung innerhalb des ersten Gewebeabschnitts vor einem Festklemmen des ersten Abschnitts und innerhalb des zweiten Gewebeabschnitts vor einem Festklemmen des zweiten Abschnitts.

13. Chirurgisches Instrument nach Anspruch 12, dadurch gekennzeichnet, daß der Stent aus einem bioabsorbierbaren Material besteht, so daß er nach einem Abnehmen der Klemmvorrichtung in den verschweißten Gewebeabschnitten zurückbleiben kann.

14. Chirurgisches Instrument nach einem beliebigen der vorangehenden Ansprüche, dadurch gekennzeichnet, daß jeder der besagten Arme im wesentlichen senkrecht an einem Achsbolzen angebracht ist, und eines der besagten Armpaare in Längsrichtung am Achsbolzen entlang auf das andere Armpaar zu verschiebbar ist, um einen der festgeklemmten Gewebeabschnitte relativ zum anderen zu bewegen.

## Revendications

1. Appareil chirurgical destiné au soudage par laser des extrémités en aboutement de deux sections corporelles tissulaires tubulaires creuses (A, B), l'appareil comprenant un moyen (T) servant à rapprocher les première et seconde extrémités de section tissulaire et des moyens (30, 50) servant à transmettre une énergie de laser à la jointure en vue de souder ensemble les première et seconde extrémités de section tissulaire, l'appareil étant caractérisé en ce que le moyen de rapprochement comprend :
un premier moyen de serrage (12, 16) définissant un premier canal (107, 147) destiné à recevoir et à saisir circonférentiellement la première section tissulaire, et
un second moyen de serrage (10, 14) définissant un second canal (127, 167) servant à recevoir et à saisir circonférentiellement la seconde section tissulaire, et
un moyen (203) supportant à la fois le premier moyen de serrage et le second moyen de serrage pour déplacer l'un desdits moyens de serrage vers l'autre moyen de serrage pour amener les première et seconde extrémités des sections tissulaires en relation d'aboutement compressif pour former une jointure annulaire.

2. Appareil chirurgical selon la revendication 1, dans lequel ledit moyen servant à serrer ladite première section tissulaire comprend une première paire de bras opposés (12, 16) et dans lequel ledit moyen pour serrer ladite seconde section tissulaire comprend une seconde paire de bras opposés (10, 14), l'un au moins desdits bras de chacune desdites paires étant relié en pivotement audit bras opposé et pouvant être déplacé d'une position ouverte distante dudit bras opposé jusqu'à une position fermée recouvrant ledit bras opposé.

3. Appareil chirurgical selon la revendication 2, comprenant en outre un axe (20) et un couvercle (203) monté sur une extrémité dudit axe, lesdits bras étant montés quasiment perpendiculaires audit axe, dans lequel la rotation dudit couvercle amène l'une desdites paires de bras à coulisser longitudinalement le long dudit axe vers ladite autre paire de bras.

4. Appareil chirurgical selon la revendication 3, dans lequel un premier bras de ladite première paire de bras est fixé de manière fixe audit axe et les deux bras de ladite seconde paire de bras sont montés avec faculté de pivotement sur ledit axe, ledit axe étant aligné quasiment parallèle à un axe longitudinal desdites sections serrées destinées à être soudées.

5. Appareil chirurgical selon l'une quelconque des revendications précédentes, comprenant en outre un moyen d'attache (22) servant à maintenir lesdits bras opposés de chaque dite paire de bras dans ladite position fermée.

6. Appareil chirurgical selon la revendication 5, dans lequel ledit moyen d'attache comprend un élément de retenue monté avec faculté d'inclinaison sur l'un desdits bras de chacune desdites paires de bras, ledit élément de retenue comportant une partie dépouillée conçue pour recouvrir ledit bras opposé dans ladite position fermée et comprenant en outre un dispositif de fixation servant à serrer ledit élément de retenue contre lesdits bras en vue de fixer lesdits bras opposés.

7. Appareil chirurgical selon l'une quelconque des revendications précédentes, dans lequel chacun desdits bras opposés comporte une partie dirigée vers l'intérieur (107), chaque dite partie de chaque dit bras comportant un évidement (147) qui y est formé pour coopérer avec ladite partie dirigée vers l'intérieur dudit bras opposé lorsque lesdits bras se trouvent dans la position fermée pour ainsi former un canal destiné à recevoir la section tissulaire respective.

8. Appareil chirurgical selon l'une quelconque des revendications précédentes, dans lequel ledit moyen de transmission d'énergie de laser comprend un logement (1) conçu pour être placé sur ledit moyen de serrage, ledit logement comportant une pluralité d'éléments conducteurs de la lumière (30, 50) qui y sont disposés, ladite extrémité distale desdits éléments conducteurs de la lumière aboutissant près de ladite jointure et ladite extrémité proximale desdits éléments conducteurs de la lumière étant conçue pour être reliée à une source de laser externe.

9. Appareil chirurgical selon la revendication 8, dans lequel ledit logement comprend une première section de logement (3) conçue pour être placée sur une première surface desdits moyens de serrage et une seconde section de logement (5) conçue pour être placée sur une surface opposée desdits moyens de serrage.

10. Appareil chirurgical selon la revendication 8 ou 9, dans lequel lesdits éléments conducteurs de la lumière s'étendent vers ladite jointure en un agencement qui est conçu pour transmettre simultanément l'énergie du laser à quasiment la circonférence entière de la jointure.

11. Appareil chirurgical selon l'une quelconque des revendications précédentes, dans lequel lesdits bras sont montés sur un axe (20) et comprenant en outre un montant central (18) disposé sur ledit axe entre lesdites paires de bras et un moyen servant à serrer lesdites première et seconde sections de logement contre ledit montant central, dans lequel ledit moyen de serrage comprend une vis s'étendant à travers une ouverture dans chaque dite section de logement dans laquelle une rotation de ladite vis déplace ladite vis vers l'intérieur en direction dudit montant central pour être pressée contre ledit montant.

12. Appareil chirurgical selon l'une quelconque des revendications précédentes, comprenant une sonde destinée à être placée à l'intérieur de la première section tissulaire avant serrage de la première section et à l'intérieur de la seconde section tissulaire avant serrage de la seconde section.

13. Appareil chirurgical selon la revendication 12, dans lequel la sonde est constituée d'un matériau bioabsorbable de sorte à pouvoir demeurer dans les sections tissulaires soudées après enlèvement du clamp.

14. Appareil chirurgical selon l'une quelconque des revendications précédentes, dans lequel chacun desdits bras est monté quasiment perpendiculaire à un axe et dans lequel l'une desdites paires de bras est apte à coulisser vers l'autre paire de bras longitudinalement suivant l'axe en vue de déplacer l'une des sections tissulaires serrées par rapport à l'autre.
